Europäisches Patentamt

European Patent Office

Office européen des brevets

(11) Publication number: **0 153 164**
**B1**

(12) **EUROPEAN PATENT SPECIFICATION**

(45) Date of publication of patent specification: **02.05.90**

(51) Int. Cl.⁵: **A 61 M 1/28**

(21) Application number: **85301011.4**

(22) Date of filing: **15.02.85**

(54) Peritoneal dialysis and compositions for use therein.

(30) Priority: **18.02.84 GB 8404299**

(43) Date of publication of application:
**28.08.85 Bulletin 85/35**

(45) Publication of the grant of the patent:
**02.05.90 Bulletin 90/18**

(84) Designated Contracting States:
**AT BE CH DE FR IT LI LU NL SE**

(56) References cited:
**EP-A-0 076 355**
**EP-A-0 115 911**
**WO-A-82/03329**
**WO-A-83/00087**

(73) Proprietor: **M.L. LABORATORIES PLC**
**Marcol House, 293 Regent Street**
**London W1R 7PD (GB)**

(72) Inventor: **Milner, Jeremiah**
**22 Albert Road**
**Cheltenham Gloucestershire (GB)**

(74) Representative: **Jukes, Herbert Lewis et al**
**Swann, Elt & Company 31 Beaumont Street**
**Oxford OX1 2NP (GB)**

# EP 0 153 164 B1

**Description**

This invention relates to peritoneal dialysis.

Our Application EP—A—0115911 published after the priority date of the present application discloses peritoneal dialysis compositions containing an osmotic agent comprising a glucose polymer mixture, said mixture including at least 15%, preferably from 20 to 50%, by weight of glucose polymers of D.P. greater than 12. (D.P. is an abbreviation of "degree of polymerisation").

Peritoneal dialysis can be used for treatment of both acute and chronic renal failure. The treatment should ideally serve to restore the composition of the blood of the patient to that which would prevail if the patient's kidneys were functioning normally, and to maintain the blood in such a state. In the present state of the art such ideal results are not achievable and are probably not likely to be achieved in the near future, especially as some of the normal kidney functions, for example synthesis of hormones and enzymes, are of such a nature that dialysis of any kind cannot be expected to compensate for loss of these functions. However, even with its recognized imperfections, the established technique of carrying out peritoneal dialysis using dextrose as the osmotic agent has been of great value and any improvement over it is of potential importance.

WO—A—8300087 discloses a peritoneal dialysis solution containing glucose polymers having an average D.P. of 4 to 10.

As explained in our Application No. EP—A—0 115 911 the use of an osmotic agent which is a glucose polymer mixture including at least 15% by weight of glucose polymers of D.P. greater than 12 has advantages over the use of dextrose as the osmotic agent. We have now found that for some purposes it is advantageous to use an osmotic agent which is a glucose polymer mixture containing more than 50% by weight of glucose polymers of D.P. greater than 12.

Clinical tests carried out with a peritoneal dialysis solution containing, as the osmotic agent, a glucose polymer mixture of the composition set out in Example 5 of our Application No. EP—A—0 115 911 demonstrated that the rates of extraction of urea, creatinine, uric acid, and phosphates were significantly higher than those achievable using dextrose as the osmotic agent. Insulin secretion did not rise, blood glucose was substantially normal, and only small quantities of amino acids passed into the peritoneum. These desirable results were achieved in conjunction with withdrawal of water from the blood at a rate that could be varied to meet the needs of the patient by varying the initial concentration of the glucose polymer mixture in the dialysing solution.

Clearly, the use of such a glucose polymer mixture in peritoneal dialysis represents a considerable step forward in the art. Nevertheless, it was not reasonably to be expected that the dialysis solutions containing these glucose polymer mixtures as the osmotic agent would be equally good for all clinical purposes. In particular, the performance of a dialysis solution can in general be regarded as less critical when it is used in intermittent peritoneal dialysis, for example in treatment of acute renal failure, than when it is used in continuous peritoneal dialysis, because a patient undergoind the latter treatment may be subject to a cumulative effect of any shortcoming of the treatment.

We have observed that when using a solution containing 10% or more by weight of the glucose polymer composition of Example 5 of our Application No. EP—A—0115911 there is, in some patients, a significant loss of oligosaccharides from the peritoneum. (Oligosaccharides are herein defined as glucose polymers of D.P. from 2 to 10 inclusive). This was reflected in raised levels of maltose and maltotriose in the serum. On discontinuing dialysis it was found that the serum maltose level fell only slowly over a period of eighteen hours, suggesting that there had been storage of oligosaccharides. Assessment of the behaviour of the glucose polymers of a molecular weight higher than that of the oligosaccharides, by measurement of the concentrations of these in both the serum and the peritoneum, revealed that there was good retention of these in the peritoneum with minimal appearance of them in the serum.

Although the dialysis compositions of our Application No. EP—A—0115911 are plainly superior to similar compositions containing dextrose as the osmotic agent, it is of course desirable to provide peritoneal dialysis compositions which further reduce the possibility of transfer of carbohydrate from the peritoneum to the serum.

It is an object of the present invention to provide peritoneal dialysis compositions having improved properties in this and other respects.

Accordingly, the present invention provides a peritoneal dialysis composition containing an osmotic agent comprising a glucose polymer mixture, said mixture including more than 50% by weight of glucose polymers of D.P. greater than 12. For many purposes, the mixture may contain from 50 to 90% by weight of glucose polymers of D.P. greater than 12. For the treatment of patients in respect to whom it is desirable to keep to a minimum the transfer of carbohydrate from the peritoneum to the serum it is advantageous to use a mixture containing from 75 to 100%, preferably 90 to 100%, by weight of glucose polymers of D.P. greater than 12. The average molecular weight of the polymer mixture is preferably from 15,000 to 25,000, more preferably 18,000 to 22,000 (as determined by high pressure liquid chromatography).

In the case of patients being treated by continuous peritoneal dialysis, it is particularly desirable that the content of oligosaccharides in the glucose polymer mixture should be kept at a level low enough to ensure that too great a storage of carbohydrate does not occur, due to transfer of oligosaccharides from the peritoneum to the serum. Different patients have different requirements in this respect but it is desirable

2

that for patients undergoing continuous treatment, especially those on C.A.P.D. (continuous ambulatory peritoneal dialysis) the oligosaccharide content of the glucose polymer mixture should be no higher than 10% by weight, the mixture containing from 90 to 100% by weight of glucose polymers of D.P greater than 10.

The content of free glucose in the compositions of the invention is within the discretion of the clinician. The glucose polymer mixtures commonly contain from 0—3% by weight of glucose but higher amounts may be present if the needs of the patient so dictate.

For use in peritoneal dialysis the compositions of the present invention are in the form of an aqueous solution which preferably contains from 2 to 10% by weight of the glucose polymer mixture, the concentration of which is selected according to the nature of the treatment involved and the needs of the patient. The upper limit of this concentration is in any case subject to keeping the viscosity of the solution low enough that it will pass easily through the smallest bore of the dialysis apparatus being used for introducing the solution into the patient's peritoneum and withdrawing it therefrom. The lower limit is dependent on the need to establish a gradient of osmotic pressure between the serum and the peritoneum sufficient to achieve the desired clearances of water and waste products from the serum but not so great as to effect an intolerably rapid rate of withdrawal of water. Each patient may need to be prescribed for individually; however, a concentration of from about 2 to about 4% by weight appears to be suitable for most patients on C.A.P.D.

Apart from the presence of the specified glucose polymer mixture, the dialysis solution has a composition similar to that of the known dialysis solutions using dextrose as the osmotic agent, this composition being well known in the art and forming no part of the present invention.

The mode of use of the dialysis solutions according to the invention is similar to that of known dialysis solutions. The solution is infused into the peritoneum and allowed to remain there for a predetermined time, after which it is withdrawn and replaced by fresh solution. With the solutions of the present invention it has been found that the dialytic effect is maintained for a longer time than has been the case for most known dialysis solutions. This offers the possibility of achieving adequate clearances of water and waste products from the patient with administration of fewer infusions than has been required in the past, a matter of particular importance in C.A.P.D. where the less frequently the dialysis solution has to be changed the less chance there is of infection (which occurs chiefly during replacement of spent solution by fresh solution).

One of the advantages of the osmotic agents used in the present invention is that they are capable of sustaining an osmotic pressure gradient across the peritoneal membrane for longer periods than has been feasible when using dextrose as the osmotic agent. Because of the rapid decay of the osmotic pressure gradient when using dextrose, it has been necessary to use dialysis solutions having a relatively high initial osmolality. The osmolality of human blood is of the order of 300 mOsm/kg. In oder to minimise stress on the peritoneal membrane it is desirable that the osmolality of the dialysis solution should exceed that of the patient's blood by as little as is compatible with achieving satisfactory dialysis. The compositions of the present invention make it possible to achieve satisfactory dialysis with an initial osmolality of the dialysis solution considerably less than when the osmotic agent is dextrose or when it is a glucose polymer containing a substantial proportion of oligosaccharides, and at the same time to sustain dialysis for longer periods than with previously used dialysis solutions.

In favourable cases it is possible for peritoneal dialysis solutions of the present invention to be allowed usefully to remain in the peritoneum for as long as eight hours. The dialy regimen for a patient suffering from chronic renal failure and being treated by continuous peritoneal dialysis then involves three exchanges per day. Each infusion is of two litres in volume so the total infusion per day is six litres. The total amount of fluid withdrawn per day is 7.2 litres, corresponding to a clearance of 5 ml/minute, for components in respect to which equilibrium is achieved between dialysate and plasma.

When treating a patient suffering from acute renal failure (or suffering from poisoning from a drug overdose, as will be discussed below), it is feasible to establish a regimen involving infusion of three litres per hour, withdrawing about four litres at the end of each hourly period, corresponding to a clearance of nearly 70 ml/minute. This is lower than the clearance rate which can be achieved by haemoperfusion, which is of the order of 100 ml/minute. However, haemoperfusion cannot be continued for very long; in some cases a period of up to six hours is as long as can be tolerated without risk. By contrast, peritoneal dialysis can be continued for a very much longer time without such risk.

The glucose polymer mixtures used in this invention can be produced by hydrolysis of starch in known manner, followed by treatment of the mixture of glucose polymers so obtained in order to remove some or all of the glucose polymers of lower molecular weight. Preferably, a glucose polymer mixture is prepared by the process described in our Application No. EP—A—0115911 and removal of lower molecular weight polymers is then effected by known fractionation techniques, such as solvent fractionation, or separation of the polymers with the aid of permeable membranes of appropriate cutoff characteristics.

In general, the fractionation techniques employed are such as to remove mainly the lower molecular weight polymers. If desired, however, they may also be used to remove very high molecular weight polymers if this is found to be necessary in order to ensure that all of the final mixture of glucose polymers is sufficiently water-soluble for there to be no tendency for higher molecular weight polymers to precipitate from solution on standing.

3

The following Examples are given by way of illustration and without limitation.

Example 1

The glucose polymer mixture of Example 5 of our Application No. EP—A—0115911 was washed repeatedly with aqueous ethanol to remove lower molecular weight glucose polymers in the filtrate. Chromatographic analysis of the solid residue showed that after two such extractions the glucose polymer mixture contained less than 5% by weight of polymers of D.P. of 12 or less. After removal of pyrogens (by passing a solution of the glucose polymer mixture through a column of activated charcoal) this glucose polymer mxiture was suitable for use as the osmotic agent in a peritoneal dialysis composition according to the present invention.

Example 2

The glucose polymer mixture of Example 5 of our Application No. EP—A—0115911 was dissolved in water. To this solution was added 95% w/w ethanol with continuous stirring until a concentration of 55% ethanol was reached. The precipitated higher molecular weight polymers were then separated by decantation. A portion of this separated syrup was analysed and was found to have the following composition:—

| | |
|---|---|
| G1 | 0.5 |
| G2 | 1.2 |
| G3 | 1.4 |
| G4 | 1.1 |
| G5 | 1.9 |
| G6 | 3.6 |
| G7 | 2.2 |
| G8 | 0.7 |
| G9 | 0.5 |
| G10 | 0.4 |
| G11 | 0.3 |
| G12 | 0.3 |
| G13—14 | 7.2 |
| G15 and over | 79.1 |

Thus, the polymer mixture (which would be capable of use as an osmotic agent according to the invention) contained 86.3% of polymers of D.P. greater than 12 and 13.5% of polymers of from D.P. 1 to 10.

The remainder of the separated syrup was redissolved in water and re-precipitated by addition of 95% w/w ethanol, this time to achieve a concentration of 65% ethanol. The precipitate was then dissolved in water and the solution was evaporated to dryness. The composition of the glucose polymer mixture so obtained was as follows:—

| | |
|---|---|
| G1 | — |
| G2 | 0.7 |
| G3 | 0.9 |
| G4 | 0.6 |
| G5 | 0.9 |
| G6 | 2.0 |
| G7 | 1.7 |
| G8 | 0.5 |
| G9 | 0.3 |
| G10 | 0.3 |
| G11 | 0.2 |
| G12 | 0.2 |
| G13—14 | 5.8 |
| G15 and over | 86.1 |

Thus, the polymer mixture contained 91.9% of polymers of D.P. greater than 12 and 7.9% of polymers of from D.P. 2 to 10. The average molecular weight of this polymer mixture, determined by H.P.L.C., was 23,700. This glucose polymer mixture was the subject of clinical and other tests and is referred to below as "the glucose polymer mixture of Example 2".

Example 3

23Kg of the glucose polymer mixture described in Example 5 of our Application No. EP—A—0115911 was dissolved in water, the solution having a volume of 37 litres. 32.5 litres of 85% w/w ethanol was added. After allowing two phases to separate by settling over four hours, the high molecular weight lower syrup phase, containing approximately 10% of the polymers, was withdrawn and discarded. To the upper phase was added 40 litres of 85% w/w ethanol, to precipitate a further 53% of the original polymers. This syrup phase (21 litres) was diluted to 46 litres with water and reprecipitated with 75 litres of 85% w/w ethanol to yield 31% of the original glucose polymers. After two further re-precipitations, a yield of 27% of the original glucose polymers was isolated. The composition of the polymer mixture thus obtained was as follows:—

| | |
|---|---|
| G1 | 0.15 |
| G2 | 0.5 |
| G3 | 0.8 |
| G4 | 0.6 |
| G5 | 0.9 |
| G6 | 2.2 |
| G7 | 1.9 |
| G8 | 0.6 |
| G9 | 0.3 |
| G10 | 0.3 |
| G11 | 0.1 |
| G12 | 0.1 |
| G13—14 | 5.6 |
| G15 and over | 85.8 |

Thus, the polymer mixture contained 91.4% of polymers of D.P. greater than 12 and 8.25% of polymers of from D.P. 1 to 10. The average molecular weight of the polymer mixture, determined by H.P.L.C., was 19,800.

Example 4

Clinical tests were carried out by subjecting an adult male to peritoneal dialysis with two compositions. Each composition was a solution of a type conventionally used in peritoneal dialysis, the compositions differing only in the nature of the osmotic agent used. One composition (Solution A) contained 1.36% by weight of dextrose and the other composition 5% by weight of the glucose polymer mixture of Example 2 above. The results of the tests are summarised as follows:—

1. The degree of ultrafiltration was comparable for the two solutions. In each case the patient received 2.2 liters of solution. After three hours the solution was withdrawn from the patient and for Solution A the withdrawn volume amounted to 2.375 litres whereas for Solution B it amounted to 2.430 litres.

2. The levels of uric acid, phosphates, creatinine, and urea in the serum and in the dialysate were determined. After a three hour dwell of solution in the peritoneum the ratios of concentration in the dialysate to concentration in the serum were as follows:—

| | Solution A | Solution B |
|---|---|---|
| Uric acid | 0.60 | 0.89 |
| Phosphate | 0.68 | 0.91 |
| Creatinine | 0.73 | 0.96 |
| Urea | 0.94 | 1.10 |

It is clear that Solution B gives a more efficient extraction than Solution A. The fact that the concentration of urea is higher in the dialysate than in the serum is remarkable and unexpected. This effect may be due to the formation of inclusion complexes of urea with glucose polymer molecules. It is believed that higher moleuclar weight glucose polymers have helical structures when in solution and it is therefore possible that they could form such complexes.

3. The initial osmolality of Solution A was 334 mOsm/kg; that of Solution B wsa 311 mOsm/kg. After three hours the osmolality of the dialysate formed by Solution A was about 80% of the original value, whereas the osmolality of the dialysate formed by Solution B remained the same as the original value.

Example 5

Two litres of Solution B of Example 4 were introduced into the peritoneum of an adult non-diabetic male and allowed to remain there for six hours. During that time the serum of the patient was monitored to assess the levels of glucose and of total carbohydrate. The results (in mg/dl) were as follows:—

| Time (hrs) | Serum glucose | Serum carbohydrate |
|---|---|---|
| 0 | 79.5 | 140.0 |
| $\frac{1}{4}$ | 73.5 | 94.0 |
| 2 | 63.5 | 150.0 |
| 4 | 55.2 | 160.0 |
| 6 | 50.5 | 160.0 |

Thus, the dialysis was effected without unduly loading the patient with carbohydrate. It is estimated that with three infusions of Solution B per twenty-four hours the total daily carbohydrate uptake would be about 58 g. By contrast, with a regimen daily involving four infusions of a dialysis solution containing 1.36% dextrose and one infusion of a dialysis solution containing 3.86% dextrose (regimen typical of conventional peritoneal dialysis) the carbohydrate uptake can be expected to be about 136 g.

Example 6

Three solutions suitable for use in peritoneal dialysis were prepared, all of similar composition except that as the osmotic agent one solution contained 1.36% dextrose, another contained 3.86% by weight of dextrose, and another contained 5.0% by weight of the glucose polymer mixture of Example 2. The osmolality of each solution was measured with the following results:—

| Solution | Osmolality (mOsm/kg) |
|---|---|
| 1.36% dextrose | 334.4 |
| 3.86% dextrose | 482.4 |
| 5.0% of glucose polymer mixture of example 2 | 308.0 |

It is evident that the third of these solutions would in use be less useful on the peritoneal membrane than either of the other solutions.

Example 7

Two solutions suitable for use in peritoneal dialysis were prepared, both of similar composition except that one contained, as the osmotic agent, 5.0% by weight of the glucose polymer mixture of Example 2 above, whereas the other contained, as the osmotic agent, 5.0% by weight of the glucose polymer mixture of Example 5 of our Application No. EP—A—0115911. These solutions were used in peritoneal dialysis on the same patient. The total carbohydrate content was measured for each of the final dialysates after six hours. It was found that the amount of carbohydrate lost from the peritoneum with the first solution (that of the present invention) was 22.7% and with the second solution 44.9%. The carbohydrate load on the patient was therefore considerably lower for the first solution than for the second.

The nature of the carbohydrate load imposed on the patient by these two solutions is of some significance. The glucose polymer mixture used in the first solution contains 7.9% of polymers of D.P. from 2 to 10, whereas the second solution uses a glucose polymer solution containing 61.8% of polymers of from D.P. 2 to 10. When dialysis is carried out with either solution, there occurs diffusion of polymers of all molecular weights across the peritoneal membrane into the blood. In each case, the lower molecular weight polymers permeate the membrane more rapidly than do the higher molecular weight polymers. However, because of the different composition of the glucose polymer mixtures used as osmotic agents, the carbohydrate load imposed by the first solution comprises a lower proportion of lower molecular weight polymers and a higher proportion of higher molecular weight polymers than does the load imposed by the second solution.

It appears that the higher molecular weight polymers are degraded in the blood by plasma amylase to give polymers of lower molecular weight. These polymers, for example maltotrioses, are not very readily metabolised or eliminated. The concentration of such polymers in the first solution is relatively low and they can therefore be removed from the blood by diffusion through the peritoneal membrane much more rapidly than is possible when the second solution is being used for dialysis, since the second solution already contains a high concentration of such polymers. Thus, the first solution may be said to have the capability of removing its own breakdown products from the blood.

This is illustrated by observations made in the course of the comparative tests described above. During the dialysis using the first solution, the glucose levels of the serum and of the dialysate were monitored. Initially, the serum glucose level (expressed in millimoles per litre) was 4.3. During the first hour it fell to

6

4.0, during the second hour it rose to 4.4, and during the last four hours it gradually fell to 4.2. The glucose level of the dialysate (initially insignificant) rose rapidly during the first hour to 2.1 and then less rapidly over the last five hours to reach 4.4. It will be recognized that the maintenance of a substantially constant glucose level in the blood, accompanied by a continuously rising level of glucose in the dialysate is consistent with a process in which higher molecular weight polymers that have entered the blood from the dialysate being enzymatically hydrolysed to form, inter alia, glucose which is then withdrawn into the dialysate, at least until such time as the concentration of glucose in the dialysate rises to equal that in the blood. A similar process appears to take place in respect of the oligosaccharides, the build-up of which in the blood has caused problems for some patients in the past, i.e. lower molecular weight polymers are formed in the blood by hydrolysis of higher molecular weight polymers and then diffuse into the peritoneum. With regard to this, the serum maltose level of the patient was determined after six hours of dialysis. It was found that when the first solution was used the final serum maltose level was 0.15 g/l whereas for the second solution it was 1.02 g/l.

It may be mentioned that polymers of all molecular weights are able to find their way into the blood by a route other than by diffusion through the peritoneal membrane, possibly by way of the lymphatic system.

The peritoneal dialysis compositions of the present invention, and also the peritoneal dialysis compositions of our Application No. EP—A—0115911 are of value not only in the treatment of patients suffering from kidney malfunction but also in the treatment of patients suffering from toxaemia of other origins.

Accordingly, the invention also provides a method of treating a patient suffering from toxaemia caused other than by kidney malfunction, comprising effecting peritoneal dialysis by introducing into the abdominal cavity of the patient a peritoneal dialysis composition including as an osmotic agent a glucose polymer mixture containing at least 15% by weight, preferably more than 50% by weight, of glucose polymers of D.P. greater than 12.

Such toxaemia may be caused by toxins which arise from internal disorders of the body or are derived from external sources.

Various diseases and disorders give rise to toxaemia which can be treated by peritoneal dialysis using as osmotic agents the glucose polymer mixtures referred to. They include gout, hepatic encephalopathy, amino acid opathies (MSUD), neurological disorders caused by accumulation in the blood of long chain fatty acids, and cases of intractable heart failure in which an excess of water in the blood constitutes the toxin.

Toxaemia caused by toxins from sources external to the body includes cases of poisoning by overdoses of drugs, for example barbiturates, salicylates, lithium, and quinidine, and industrial or agricultural chemicals, for example the herbicide paraquat.

In considering the potential value of treatment of such toxaemia by peritoneal dialysis using, as osmotic agents, the glucose polymer mixtures referred to, it is necessary to take into account the fact that the rate of removal of a chemical from the body into the peritoneal fluid will depend on a number of factors which include:—

1. The degree of plasma protein binding of the chemical, because only molecules which are free in the plasma water will be dialysed.

2. The volume of distribution of the chemical, which determines the proportion of the total body burden of the chemical which is in the blood and is therefore immediately available for dialysis. If the volume of distribution is high (i.e. the proportion of total chemical in the blood is low) then dialysis for prolonged periods will be needed to effect a significant removal of the chemical.

Furthermore, the value of such treatment is subject to:—

(a) whether a significant rate of clearance can be achieved,

(b) whether the rate of clearance is substantial when compared with the body's normal rate of clearance (by metabolism or excretion), and

(c) whether peritoneal dialysis can be quickly and easily initiated on a patient.

With regard to (c) it appears that in most cases peritoneal dialysis could be initiated more quickly and easily than haemodialysis or haemoperfusion and probably with less risk of harming the patient. In addition, unlike haemoperfusion, it should be possible to continue with peritoneal dialysis for prolonged periods, so as to avoid rebound of the blood level of the chemical (due to influx from the tissues) that can occur when haemoperfusion has to be stopped because of such problems as loss of platelets.

As mentioned above, any binding of the chemical to plasma proteins would limit the rate of clearance of the chemical to the peritoneum because only unbound chemical is free to equilibrate with that in the dialysis solution. However, in a case where it is possible to reduce the free concentration of the chemical in the dialysis solution to a level below that in the plasma, a better clearance can be achieved. Natural or synthetic complexing agents may be considered for this purpose. For example, the inclusion of human albumin in the dialysis solution may in some cases offer the possibility of effecting improved clearance of some drugs, such as tricyclic anti-depressants which are highly bound to plasma protein.

A discussion of the potential value of peritoneal dialysis for the treatment of paraquat poisoning will serve for illustration of this aspect of the invention. (Paraquat is a trade name for the herbicide 1.1' - dimethyl - 4,4″ - dipyridium dichloride).

In the paraquat (PQ) poisoned patient an early and important event is renal failure. The time of onset and degree of renal failure determines how much PQ is retained in the body to allow the slow accumulation of toxic concentrations in the lung. The kidney lesion is reversible but the lung lesion is not and is usually the cause of death unless the patient has taken a massive dose and dies within 2—3 days. A method for removing PQ from the circulation in the interval between onset of renal failure and accumulation of toxic levels in the lung (approximately 12—18 hours in man) would be valuable. This method must be readily instituted (within 1 hour) and must be capable of being maintained for long periods (3—4 days) until renal function begins to return and the body burden of PQ has been largely eliminated.

Peritoneal dialysis with the glucose polymer mixtures referred to above represents an attractive and better alternative to haemodialysis or haemoperfusion for the removal of PQ. The purpose of the study described below was to determine whether a solution of the specified glucose polymers in the peritoneum of a dog extracts PQ from the circulation.

Experimental procedure

A 28.5 Kg greyhound dog was anaesthetized with pentobarbital. A venous cannula was inserted into one foreleg vein for collection of blood samples and into the other for administration of PQ. A bladder catheter was inserted for collection of urine. An intravenous dose of 20 mg/kg (570 mg and 116 µg of $^{14}$C-PQ—51.5 µCi) of paraquat was administered over a 5 minute period. Urine samples were collected every hour, volume was recorded and aliquots were taken for measurement of radioactivity. Blood samples were taken every 30 minutes, plasma was separated and counted. Two hours after the administration of the paraquat 33 ml/kg (1180 ml) of a peritoneal dialysis solution (of conventional composition except that instead of dextrose the solution contained, as the osmotic agent, 10% by weight of the mixture of glucose polymers of Example 5 of our Application No. EP—A—0115911) was introduced into the peritoneum through a catheter. Samples of the dialysis fluid were taken at intervals for two hours. Two hours after the introduction of the peritoneal dialysis fluid, it was removed and the experiment was terminated.

Results

The experiment was completed according to the protocol. However, the intravenous dose of paraquat did not induce renal failure within 2 hours as was expected. The renal clearance of paraquat did not decline (Table 1). A larger dose with a longer time period would evidently be needed to cause renal failure. Nevertheless, this study clearly shows that paraquat readily passes from the blood into the dialysis fluid in the peritoneum (Table 2). The rate of rise of the concentration of paraquat in the peritoneum was rapid at first so that half equilibration with the plasma concentration was achieved in 40 minutes. A similar result was obtained in a further study with another dog. If we assume that half-equilibration with plasma concentration is achieved in 40 minutes in a poisoned patient with renal failure and that a total of 4 litres of fluid is introduced into the peritoneum then by changing the fluid at intervals of 40 minutes to 1 hour a clearance of paraquat of 2000/40 ml per min or 50 ml/min could be achieved. This is far greater than renal clearance in the poisoned patient and is comparable to values obtained with haemodialysis or haemoperfusion (60—100 ml/min).

TABLE 1
Renal clearance of paraquat

| Time (h) | Clearance (ml/min) |
|---|---|
| 1.5 | 64.3 |
| 2.5 | 48.4 |
| 3.5 | 56.0 |

TABLE 2
Peritoneal dialysis of paraquat
The appearance of paraquat in the dialysate

| Time (min) | Paraquat (µg/ml) |
|---|---|
| 0 | 0 |
| 15 | 2.89 |
| 30 | 3.67 |
| 47 | 4.72 |
| 60 | 5.73 |
| 87 | 6.06 |
| 120 | 6.40* |

*(Plasma paraquat 8.91 µg/ml)

## Claims

1. The use in the preparation of a peritoneal dialysis composition of an osmotic agent comprising a glucose polymer mixture, said mixture being derived from a starch hydrolysate and containing more than 50% by weight of glucose polymers of D.P. greater than 12.

2. The use of Claim 1 wherein said mixture contains up to 90% by weight of glucose polymers of D.P. greater than 12.

3. The use of Claim 1 wherein said mixture contains from 75 to 100% by weight of glucose polymers of D.P. greater than 12.

4. The use of Claim 3 wherein said mixture contains from 90 to 100% by weight of glucose polymers of D.P. greater than 12.

5. The use of any of Claims 1 to 4 wherein said mixture contains no more than 10% by weight of glucose polymers of D.P. of from 2 to 10.

6. The use of any of Claims 1 to 5 wherein said mixture contains from 0.0 to 3.0% of glucose.

7. The use of any of Claims 1 to 6 in the form of a peritoneal dialysis solution containing from 2 to 10% w/v of said glucose polymer mixture.

8. The use of Claim 7 wherein said solution contains from 2 to 4% w/v of said glucose polymer mixture.

9. The use of any of Claims 1 to 8, wherein the average molecular weight of the polymer mixture is from 15,000 to 25,000 or from 18,000 to 22,000.

## Patentansprüche

1. Verwendung eines osmotischen Mittels aus einer Glukosepolymermischung, wobei die Mischung auf ein Stärkehydrolysat zurückgeht und mehr als 50 Gew.-% Glukosepolymere mit einem Polymerisationsgrad (D.P.) von mehr als 12 enthält, zur Herstellung einer peritonealen Dialysezubereitung.

2. Verwendung nach Anspruch 1, wobei die Mischung bis zu 90 Gew.-% Glukosepolymere mit einem Polymerisationsgrad von mehr als 12 enthält.

3. Verwendung nach Anspruch 1, wobei die Mischung 75 bis 100 Gew.-% Glukosepolymere mit einem Polymerisationsgrad von mehr als 12 enthält.

4. Verwendung nach Anspruch 3, wobei die Mischung 90 bis 100 Gew.-% Glukosepolymere mit einem Polymerisationsgrad von mehr als 12 enthält.

5. Verwendung nach einem der Ansprüche 1 bis 4, wobei die Mischung nicht mehr als 10 Gew.-% Glukosepolymere mit einem Polymerisationsgrad von 2 bis 10 enthält.

6. Verwendung nach einem der Ansprüche 1 bis 5, wobei die Mischung 0,0 bis 3,0% Glukose enthält.

7. Verwendung nach einem der Ansprüche 1 bis 6 in Form einer peritonealen Dialyselösung, die 2 bis 10% (Gewicht/Volumen) der Glukosepolymermischung enthält.

8. Verwendung nach Anspruch 7, wobei die Lösung 2 bis 4% (Gewicht/Volumen) der Glukosepolymermischung enthält.

9. Verwendung nach einem der Ansprüche 1 bis 8, wobei das durchschnittliche Molekulargewicht der Polymermischung 15000 bis 25000 oder 18000 bis 22000 beträgt.

## Revendications

1. Utilisation, dans la préparation d'une composition pour dialyse péritonéale, d'un agent osmotique comprenant un mélange de polymères de glucose, ledit mélange étant dérivé d'un hydrolysat d'amidon et contenant plus de 50% en poids de polymères de glucose de degré de polymérisation supérieur à 12.

2. Utilisation suivant la revendication 1, dans laquelle ledit mélange contient jusqu'à et y compris 90% en poids de polymères de glucose de degré de polymérisation supérieur à 12.

3. Utilisation suivant la revendication 1, dans laquelle ledit mélange contient 15 à 100% en poids de polymères de glucose de degré de polymérisation supérieur à 12.

4. Utilisation suivant la revendication 3, dans laquelle ledit mélange contient 90 à 100% en poids de polymères de glucose de degré de polymérisation supérieur à 12.

5. Utilisation suivant l'une quelconque des revendications 1 à 4, dans laquelle ledit mélange ne contient pas plus de 10% en poids de polymères de glucose de degré de polymérisation ayant une valeur de 2 à 10.

6. Utilisation suivant l'une quelconque des revendications 1 à 5, dans laquelle ledit mélange contient 0,0 à 3,0% de glucose.

7. Utilisation suivant l'une quelconque des revendications 1 à 6, sous la forme d'une solution pour dialyse péritonéale contenant 2 à 10% en poids/volume dudit mélange de polymères de glucose.

8. Utilisation suivant la revendication 7, dans laquelle ladite solution contient 2 à 4% en poids/volume dudit mélange de polymères de glucose.

9. Utilisation suivant l'une quelconque des revendications 1 à 8, dans laquelle le poids moléculaire moyen du mélange de polymères va de 15 000 à 25 000 ou de 18 000 à 22 000.